# EUROPEAN PATENT SPECIFICATION

(11) **EP 2 684 551 B1**
(45) Date of publication and mention of the grant of the patent: **06.10.2021**
(21) Application number: 12755195.0
(22) Date of filing: 20.01.2012
(51) Int. Cl.: A61J 1/10, A61M 5/142, A61K 38/42

(54) **SYSTEM FOR DELIVERING OXYGEN CARRIER, OXYGENATION DEVICE FOR OXYGEN CARRIER, AND HOUSING FOR OXYGEN CARRIER**
SYSTEM ZUR AUSGABE EINES SAUERSTOFFTRÄGERS, OXYGENIERUNGSVORRICHTUNG FÜR DEN SAUERSTOFFTRÄGER UND GEHÄUSE FÜR DEN SAUERSTOFFTRÄGER
SYSTÈME POUR DISTRIBUER UN TRANSPORTEUR D'OXYGÈNE, DISPOSITIF D'OXYGÉNATION POUR TRANSPORTEUR D'OXYGÈNE, ET BOÎTIER POUR TRANSPORTEUR D'OXYGÈNE

(30) Priority: 09.03.2011 JP 2011051943; 09.03.2011 JP 2011051950; 11.03.2011 JP 2011054842; 11.03.2011 JP 2011054843
(43) Date of publication of application: 15.01.2014
(73) Proprietor: Terumo Kabushiki Kaisha, Tokyo 151-0072 (JP)
(72) Inventor: ISHIZUKA, Takanobu, Ashigarakami-gun Kanagawa 259-0151 (JP); TOKUE, Shinichi, Ashigarakami-gun Kanagawa 259-0151 (JP); KASUKAWA, Hiroaki, Ashigarakami-gun Kanagawa 259-0151 (JP); KANEDA, Shinichi, Ashigarakami-gun Kanagawa 259-0151 (JP); GOTO, Hiroshi, Ashigarakami-gun Kanagawa 259-0151 (JP); KUROSAKI, Yasuo, Ashigarakami-gun Kanagawa 259-0151 (JP); MORIMOTO, Katsumi, Ashigarakami-gun Kanagawa 259-0151 (JP)
(74) Representative: TBK
(86) International application number: PCT/JP2012/051187
(87) International publication number: WO 2012/120927

(56) References cited:
- JP-A- 61 025 557
- JP-A- 2004 538 264
- JP-A- 2006 104 069
- JP-A- 2010 509 353
- US-A- 5 827 222
- US-A1- 2003 065 149
- US-A1- 2008 069 771
- US-A1- 2010 035 798

## Description

### Technical Field

The present invention relates to a system for delivering an oxygen carrier into a tissue of a living organism according to the preamble of claim 1. Particularly, the invention relates to a system for delivering an oxygen carrier by which an oxygenated oxygen carrier can be transarterially delivered to ischemic tissue generated by a thrombus or an embolus or to hypoxic tissue, selectively and efficiently.

### Background Art

Investigations have been made into utilization of hemoglobin, which is a safe and effective source of oxygen, as a blood substitute or a therapeutic agent for pathemas in which oxygen should be supplied to a local hypoxic tissue, such as ischemic lesions in the brain or cardiac muscle, tumor tissues, and peripheral tissues brought into a circulatory insufficiency state due to diabetes or the like. As an oxygen carrier capable of supplying oxygen in these pathemas, it has been investigated to use a hemoglobin solution obtained by a method in which membrane components such as erythrocyte membrane (stroma) are removed from animal or human red blood cells to prepare stroma-free hemoglobin (SFH), followed by applying chemical modification such as crosslinking or polymerization to the stroma-free hemoglobin. Also, utilization has been investigated of an artificial oxygen carrier (artificial red blood cells) obtained by taking the stroma-free hemoglobin into liposomes. For example, document JP 2009-131672 A describes encapsulation of hemoglobin into liposome capsules, followed by modification of the outer surfaces of the liposome capsules with a hydrophilic modifying group to obtain a preparation (liposome encapsulated hemoglobin (LEH)). It is described in the document that in the preparation, the in vivo half-life of hemoglobin is prolonged as compared with free hemoglobin, and the capacity to carry oxygen to peripheral tissues is enhanced.

The oxygen carrying capacity of hemoglobin is owing to reversible binding between hemoglobin and oxygen molecule (reversible oxygenation process). In the reversible oxygenation process, while hemoglobin with ferrous ion state of heme iron (heme iron (II)) has an oxygen combining capacity, the hemoglobin itself will in the presence of oxygen be gradually oxidized (converted into methemoglobin form) to be oxidized type hemoglobin (methemoglobin). The methemoglobin, which is hemoglobin with ferrous ion state of heme iron (heme iron (III)), does not have an oxygen combining capacity.

During storage, therefore, conversion of hemoglobin to methemoglobin must be restrained, in order to maintain the oxygen carrying capacity. In this connection, it is known that since the oxidation reaction of heme iron (II) would not easily proceed in a deoxygenated liposome encapsulated hemoglobin (LEH) preparation (hemoglobin in the state where each heme iron is not combined with oxygen), preservation of the liposome preparation in the deoxygenated type form is useful.

Documents US 2008/069771 A1 and US 5 827 222 A both disclose generic systems for delivering an oxygen carrier having a housing in which a hemoglobin-based oxygen carrier is housed in a deoxygenated state. Furthermore, they have an oxygenation part for oxygenating the deoxygenated oxygen carrier, and a catheter as a long element which can be inserted into a living organism and can release the oxygenated oxygen carrier through a lumen that is formed inside thereof.

### Summary of Invention

### Technical Problem

In the case of administering an oxygen carrier such as liposome encapsulated hemoglobin (LEH) into a blood vessel for the purpose of carrying oxygen to ischemic or hypoxic tissues, the administered oxygen carrier cannot be wholly delivered to the ischemic tissue. In addition, detachment of oxygen from the oxygen carrier may occur before the administered oxygen carrier reaches the ischemic tissue. Therefore, it is desired to enhance the efficiency in carrying oxygen to the ischemic tissue.

The present invention has been made in order to solve the above-mentioned problem. Accordingly, it is an object of the present invention to provide a system for delivering an oxygen carrier by which oxygen can be efficiently delivered to an ischemic tissue by use of an oxygen carrier.

### Technical Solution

In order to attain the above object, the present invention discloses a system for delivering an oxygen carrier according to claim 1.

### Advantageous Effects

The system for delivering an oxygen carrier according to the one mode of the present invention has the long element which can be inserted into an living organism and can supply the oxygenated oxygen carrier through the lumen that is formed inside thereof. By the system, therefore, the oxygen carrier can be supplied to the target site selectively and while restraining dissociation of oxygen therefrom. Consequently, oxygen can be efficiently supplied to ischemic or hypoxic tissues by directly sending the oxygen carrier to the ischemic tissue or the local tissue.

Where the oxygenation part is provided in a transport path for the oxygen carrier that is located between the housing and the long element, the oxygen carrier can be oxygenated just before administration to a living organism. Therefore, the oxygen carrying capacity can be displayed as effectively as possible.

Where the oxygenation part achieves by mixing oxygen into the oxygen carrier present in the inside of the housing, the oxygen carrier can be easily oxygenated in the inside of the housing.

Where the housing includes an oxygen-impermeable material for blocking the housed oxygen carrier from oxygenation by external oxygen, the oxygen carrier can be stored for a long time while restraining the change thereof into a methemoglobin type form. In addition, the change of the oxygen carrier into a methemoglobin type form in the inside of the housing can also be restrained at the time of use.

### Brief Description of Drawings

[FIG. 1]

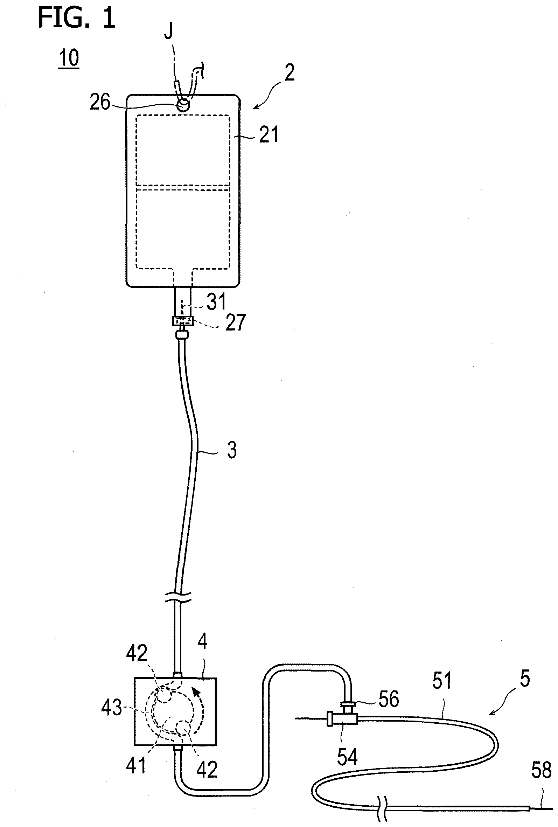

   FIG. 1 is a plan view of a system for delivering an oxygen carrier according to a first embodiment.
[FIG. 2]

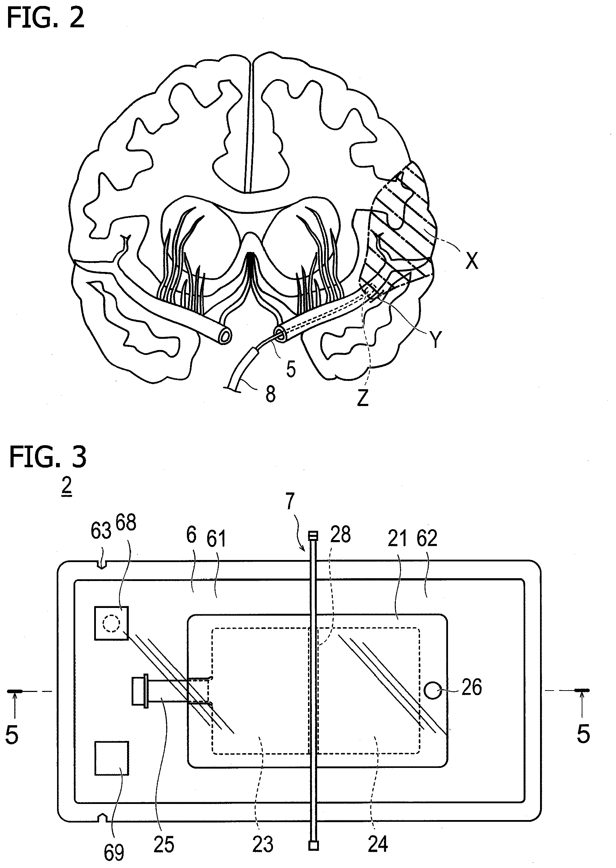

   FIG. 2 is a schematic view for illustrating a thrombosed site and an ischemic tissue in cerebral infarction as an applicable disease.
[FIG. 3]
   FIG. 3 is a plan view of an example of a housing.
[FIG. 4]

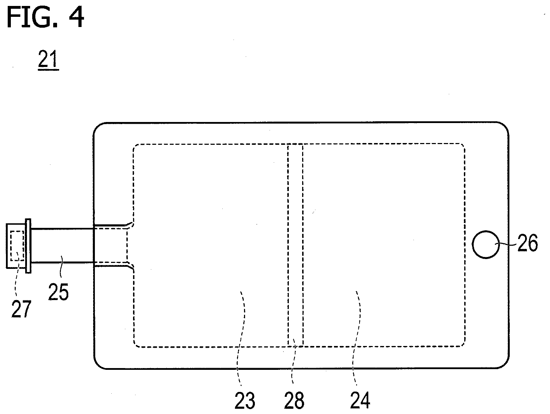

   FIG. 4 is a plan view of an example of a housing pack.
[FIG. 5]

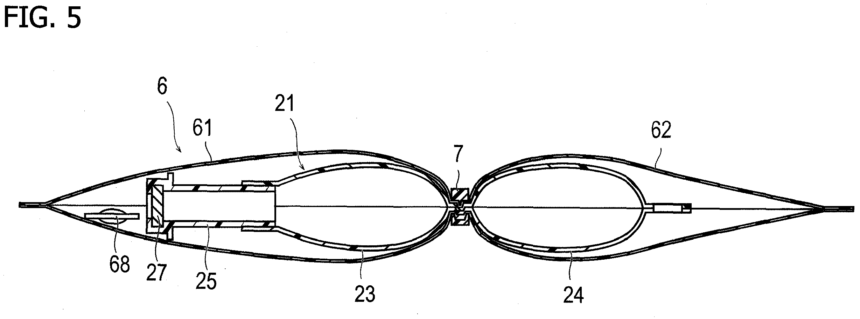

   FIG. 5 is a sectional view taken along line 5-5 of FIG. 3.
[FIG. 6]

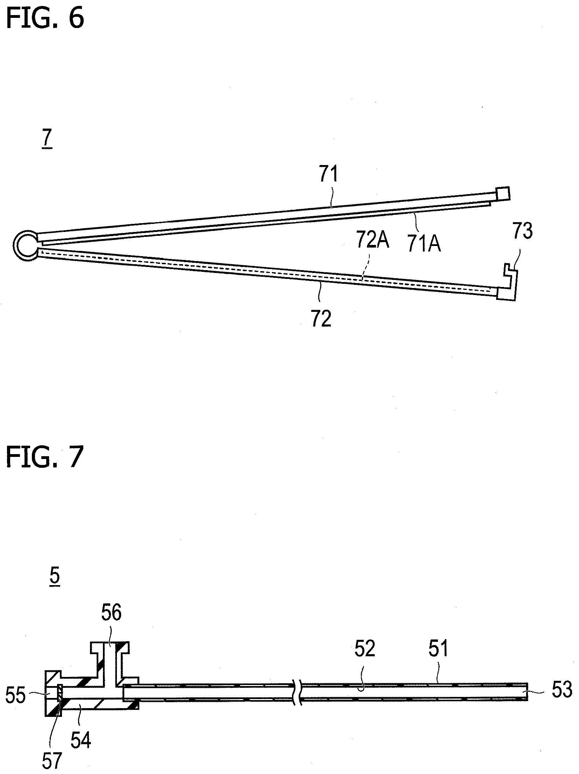

   FIG. 6 is a plan view of a clip.
[FIG. 7]
   FIG. 7 is a sectional view of a microcatheter.
[FIG. 8]

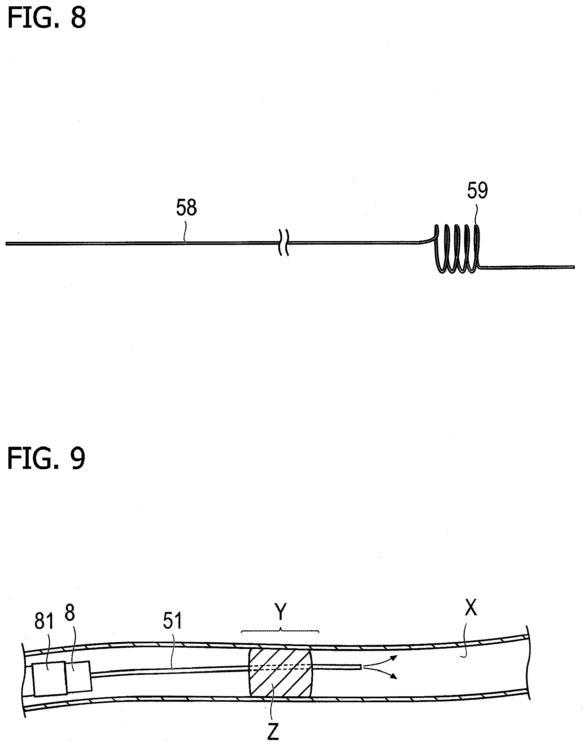

   FIG. 8 is a plan view of a retriever, as an example of a device for removing a thrombus.
[FIG. 9]
   FIG. 9 is an illustration of the supply of an oxygen carrier to an ischemic tissue by the system for delivering an oxygen carrier, as a case of local delivery.
[FIG. 10]

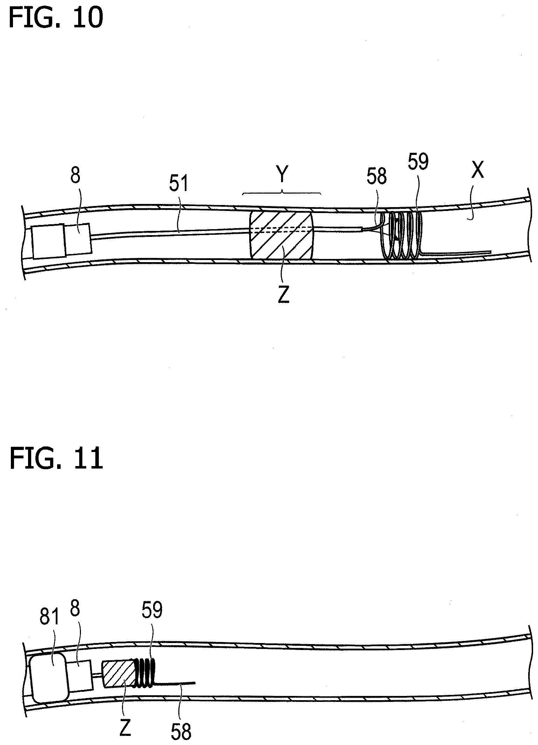

   FIG. 10 is an illustration of the insertion of a retriever to a thrombosed site by the system for delivering an oxygen carrier, in an ischemic lesion as a case of an applicable disease.
[FIG. 11]
   FIG. 11 is an illustration of the removal of a thrombus by the system for delivering an oxygen carrier, in the ischemic lesion.
[FIG. 12]

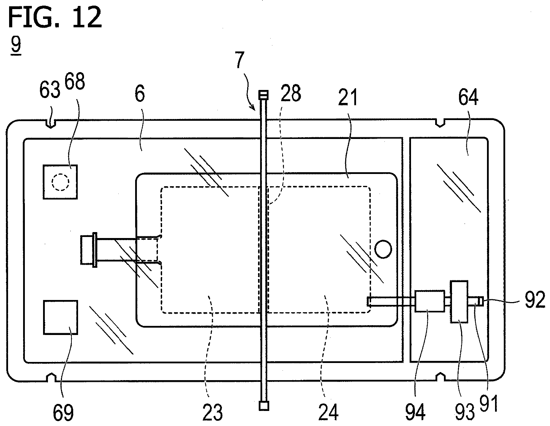

   FIG. 12 is a plan view of another example of the housing in the first embodiment.
[FIG. 13]

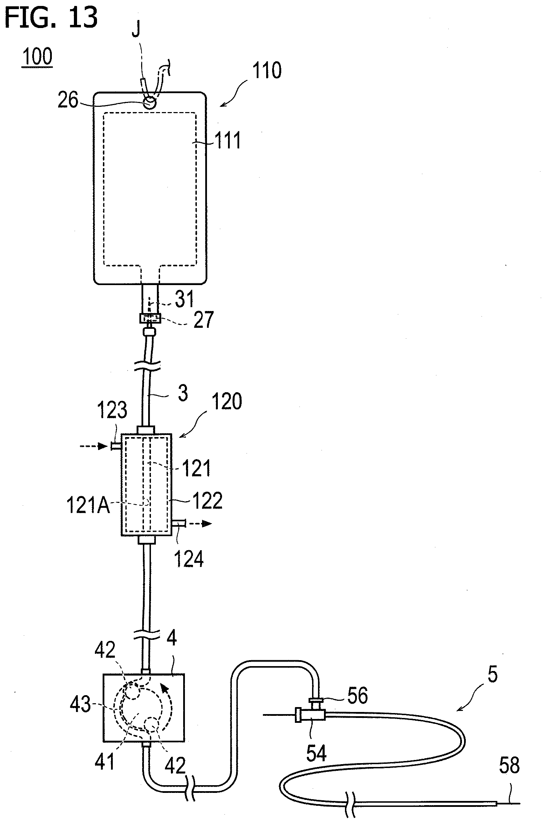

   FIG. 13 is a plan view of a system for delivering an oxygen carrier.
[FIG. 14]

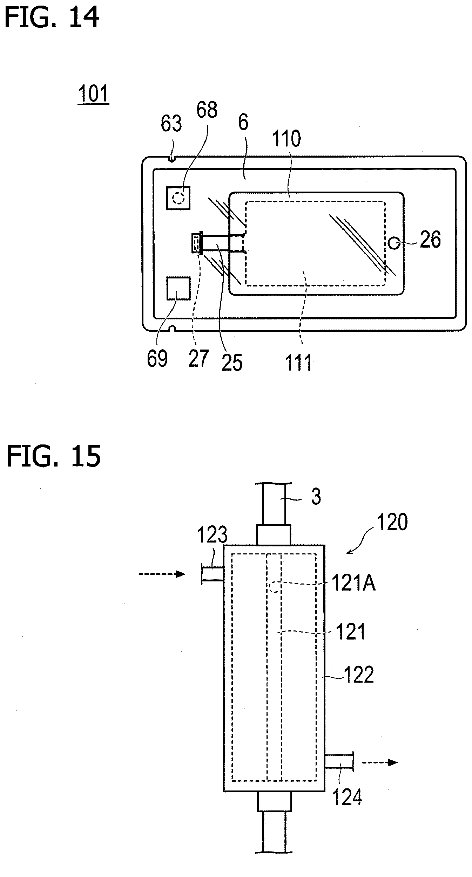

   FIG. 14 is a plan view of an example of a housing.
[FIG. 15]
   FIG. 15 is a plan view of an example of an oxygenation device for an oxygen carrier.
[FIG. 16]

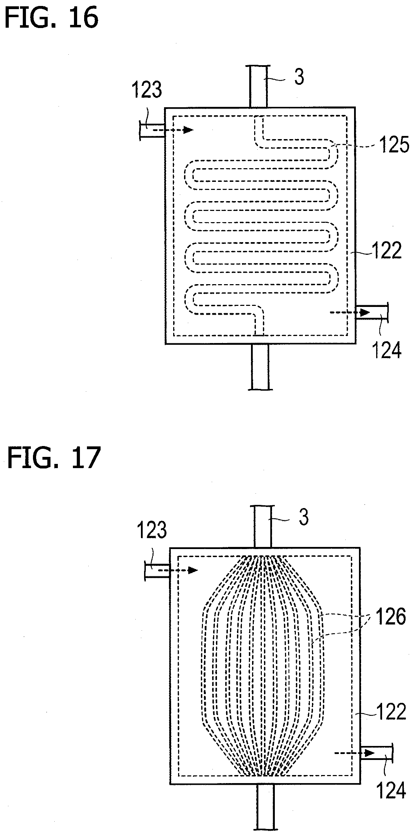

   FIG. 16 is a plan view of another example of the oxygenation device for an oxygen carrier.
[FIG. 17]
   FIG. 17 is a plan view of a further example of the oxygenation device for an oxygen carrier.
[FIG. 18]

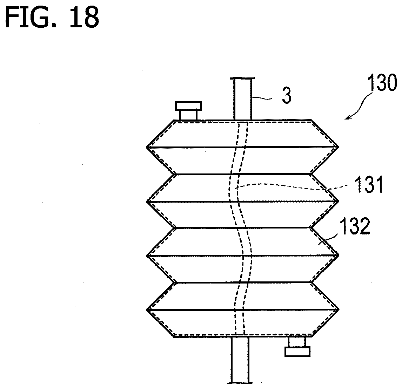

   FIG. 18 is a plan view of yet another example of the oxygenation device for an oxygen carrier.
[FIG. 19]

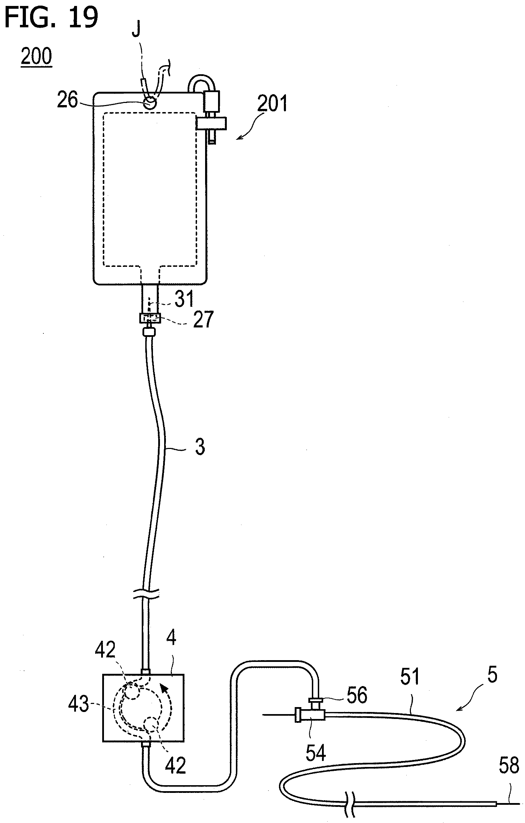

   FIG. 19 is a plan view of a system for delivering an oxygen carrier.
[FIG. 20]

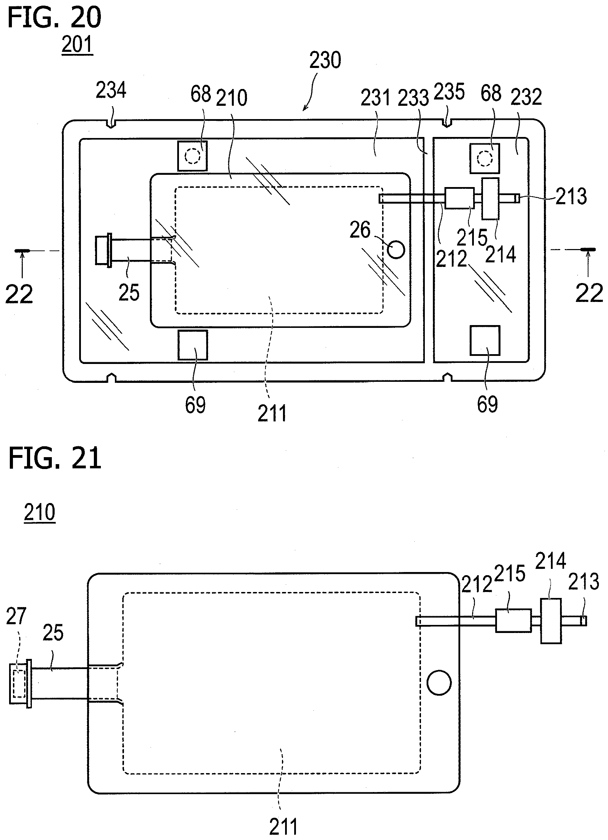

   FIG. 20 is a plan view of a housing for an oxygen carrier.
[FIG. 21]
   FIG. 21 is a plan view of a housing pack.
[FIG. 22]

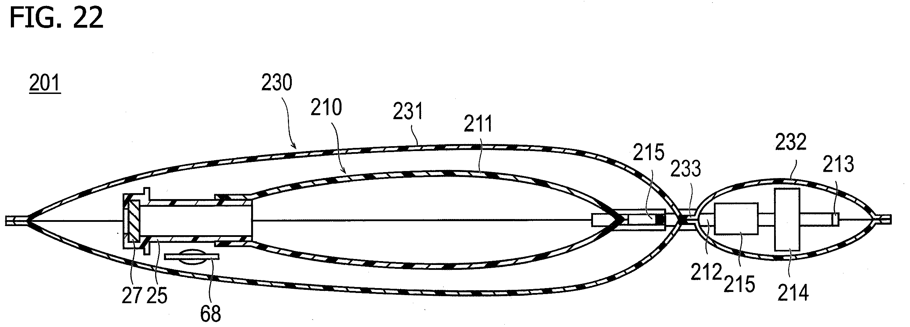

   FIG. 22 is a sectional view taken along line 22-22 of FIG. 20.
[FIG. 23]

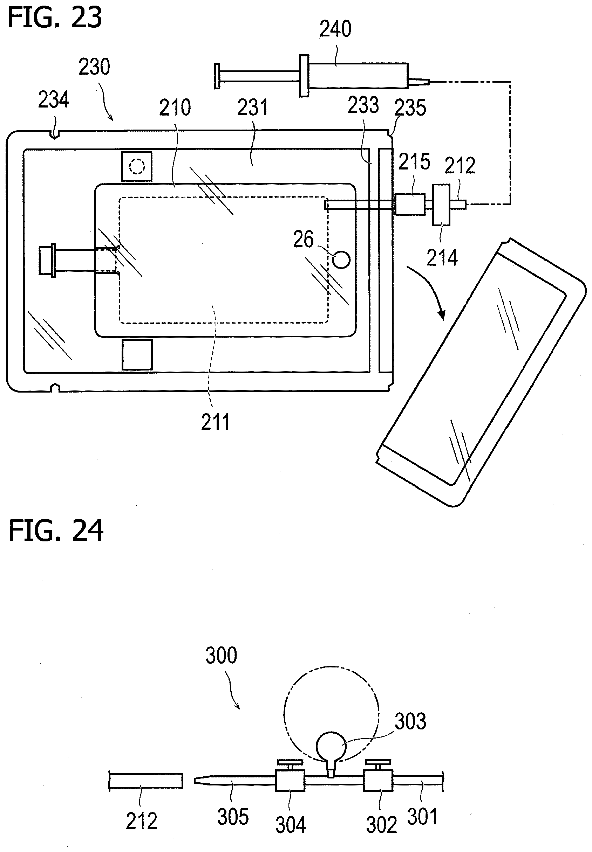

   FIG. 23 is a plan view showing a case of injecting oxygen into a housing part for a carrier via an injection tube.
[FIG. 24]
   FIG. 24 is a plan view of a modification of an oxygen supply amount control part.
[FIG. 25]

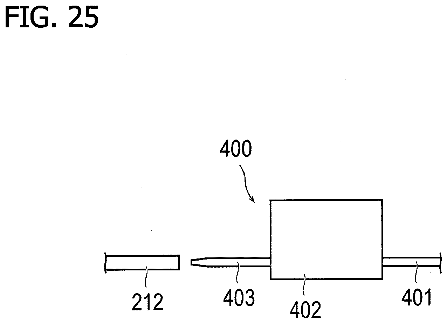

   FIG. 25 is a plan view of another modification of the oxygen supply amount control part.

### Modes for Carrying Out the Invention

Now, embodiments of the present invention will be described below referring to the drawings. Incidentally, the dimensional ratios in the drawings may be exaggerated for convenience of illustration, and may therefore be different from the actual ratios.

A system 10 for delivering an oxygen carrier according to the present invention (see Fig. 1) is a system by which a preliminarily deoxygenated hemoglobin-based oxygen carrier is oxygenated in an aseptic condition and, thereafter, the oxygenated oxygen carrier is selectively delivered to an ischemic tissue X (see FIG. 2) beyond a thrombosed site Y by way of a blood vessel.

As shown in FIG. 1, the system 10 for delivering an oxygen carrier includes a housing 2 (oxygen carrier housing) for housing and preserving an oxygen carrier, a transport tube 3 for transporting the oxygen carrier from the housing 2, a pump 4 for pressurizing the oxygen carrier transported through the transport tube 3, and a microcatheter (long element) 5 for introducing the pressurized oxygen carrier into a living organism.

The oxygen carrier is a hemoglobin-based one. Examples of the oxygen carrier include: a hemoglobin solution type carrier which is obtained by removing membrane components such as erythrocyte membrane (stroma) from human- or other animal-derived red blood cells to form stroma-free hemoglobin (SFH), and applying chemical modification such as crosslinking or polymerization to the stroma-free hemoglobin; and a liposome encapsulated hemoglobin (LEH) type carrier which is obtained by encapsulating the stroma-free hemoglobin in liposomes. More specific, but non-restrictive, examples of the oxygen carrier which can be used include those described in Japanese Patent Laid-open Nos. 2006-104069 and 2009-263269. Incidentally, these oxygen carriers are in general prepared as suspensions, as described in Japanese Patent Laid-open Nos. 2006-104069 and 2009-263269.

Specifically, the oxygen carrier may be prepared as follows. For example, the oxygen carrier may be prepared by a method including the steps of encapsulating stroma-free hemoglobin into liposomes, and modifying the outer surfaces of the liposomes with a hydrophilic modifying group such as polyethylene glycol (PEG). In this manner, a liposome encapsulated hemoglobin (LEH) type artificial oxygen carrier can be produced, but such a method is not restrictive; thus, the oxygen carrier can be prepared by a person skilled in the art by referring to the conventionally known findings or by combining them.

Incidentally, the oxygen carrier is not restricted to those prepared by such a method as above-mentioned. Other oxygen carriers and blood preparations may also be used insofar as they have an oxygen carrying capacity.

In the present embodiment, the liposome encapsulated hemoglobin (LEH) type oxygen carrier as described in Japanese Patent Laid-open No. 2006-104069 is used.

As shown in FIGS. 3 to 5, the housing 2 houses the oxygen carrier while maintaining a deoxygenated state of the oxygen carrier for the purpose of preserving the oxygen carrier for a long time. The housing 2 includes a housing pack 21 in which the oxygen carrier is actually housed, and a packaging member 6 which covers the housing pack 21.

The housing pack 21 includes: an oxygen carrier housing part 23 which houses the oxygen carrier and the like; an oxygen housing part 24 which houses oxygen; and a tubular body 25 which communicates with the oxygen carrier housing part 23 and serves for transporting out the oxygen carrier. The oxygen carrier housing part 23 and the oxygen housing part 24 are each formed from an oxygen-permeable film-shaped material so as to have a space in the inside thereof. The housing pack 21 is provided, in its edge portion opposite to the tubular body 25, with a hanging hole 26 by which it is hung on a hook J when put to use.

The oxygen carrier housing part 23 and the oxygen housing part 24 are made, for example, from polyethylene (PE); however, the material for these parts is not restricted to polyethylene, so long as it is permeable to oxygen. In addition, an oxygen-permeable material may be provided only at part of the oxygen carrier housing part 23 and the oxygen housing part 24.

The tubular body 25 is heat sealed (fused) or adhered to the film-shaped material so as to communicate with the oxygen carrier housing part 23. Like the oxygen carrier housing part 23 and the oxygen housing part 24, the tubular body 25 is made from polyethylene (PE), but this is not restrictive. The tubular body 25 is disposed so that one end thereof protrudes from the oxygen carrier housing part 23. Inside a protruding-side end portion of the tubular body 25, there is provided a rubber element 27 for sealing the inside space of the oxygen carrier housing part 23.

Between the oxygen carrier housing part 23 and the oxygen housing part 24, there is provided a sealing part 28 which maintains the oxygen carrier housing part 23 and the oxygen housing part 24 in a mutually isolated state and which, when a force is externally exerted thereon, permits the oxygen carrier housing part 23 and the oxygen housing part 24 to communicate with each other. The sealing part 28 can be easily formed, for example, by heat sealing (fusing) the film-shaped resin material constituting the housing pack 21 under controlled temperature and pressure. When the heat-sealed portion of the sealing part 28 is delaminated by an externally exerted force, the oxygen carrier housing part 23 and the oxygen housing part 24 are permitted to communicate with each other, whereby the oxygen carrier and oxygen are mixed with each other, resulting in oxygenation of the oxygen carrier. In other words, the housing pack 21 including the oxygen carrier housing part 23, the oxygen housing part 24 and the sealing part 28 functions also as oxygenating means for oxygenation of the oxygen carrier.

In the oxygen housing part 24, oxygen is contained in an amount sufficient for wholly oxygenating the hemoglobin contained in the oxygen carrier housed in the oxygen carrier housing part 23. Therefore, in an example of a case where 100 ml of an oxygen carrier suspension is housed in the oxygen carrier housing part 23 and where 6 g of hemoglobin is contained per 100 ml of the suspension, about 8 ml of oxygen is needed since the amount of oxygen necessary for oxygenation of 1 g of hemoglobin is about 1.35 ml. In view of this, oxygen is housed in the oxygen housing part 24 in an amount of not less than about 8 ml. In order to enhance the oxygen saturation of the oxygen carrier, it is preferable for oxygen housed in the oxygen housing part 24 to have an oxygen partial pressure higher than the atmospheric oxygen partial pressure (about 150 mmHg). It is also preferable to use pure oxygen (oxygen concentration: 100%), but this is not restrictive.

The packaging member 6, which envelopes the whole body of the housing pack 21, is formed from an oxygen-impermeable film-shaped material. The material constituting the packaging member 6 is desirably transparent so that the inside thereof can be visually checked. Examples of the oxygen-impermeable and light-transmitting material include films having a barrier resin layer of EVOH (ethylene-vinyl alcohol acetate copolymer), O-PVA (biaxially oriented polyvinyl alcohol), PVDC (vinylidene chloride copolymer) or the like, and films having a barrier layer obtained by coating a film of PET (polyethylene terephthalate) or the like with a thin film of an inorganic oxide such as silicon oxide, alumina, etc. by vapor deposition or the like. However, the material for the packaging member 6 is not restricted to these films, so long as the material is impermeable to oxygen.

As shown in FIGS. 3 and 5, the packaging member 6 is sealed in the state of covering the entirety of the housing pack 21, and the inside thereof is divided into two mutually isolated chambers, by a method in which a clip 7 is externally fitted thereto along the sealing part 28 of the housing pack 21. Specifically, the packaging member 6 is formed with a first outer package part 61 covering the oxygen carrier housing part 23 of the housing pack 21, and a second outer package part 62 covering the oxygen housing part 24 of the housing pack 21. In addition, a deoxygenating agent 68 (for example, AGELESS (registered trademark) produced by Mitsubishi Gas Chemical Co., Inc.) and an oxygen detecting agent 69 (for example, AGELESS EYE (registered trademark) produced by Mitsubishi Gas Chemical Co., Inc.) for detection of oxygen by color tone are encapsulated in the first outer package part 61, together with the oxygen carrier housing part 23. Therefore, hemoglobin contained in the oxygen carrier in the oxygen carrier housing part 23 is deoxygenated by the deoxygenating agent 68 through the oxygen-permeable film of the oxygen carrier housing part 23, or a deoxygenated state of a preliminarily deoxygenated oxygen carrier is maintained. Further, since the packaging member 6 is impermeable to oxygen, the oxygen carrier is maintained in the deoxygenated state, and the deoxygenated state can be confirmed by visual inspection based on the oxygen detecting agent 69.

As shown in FIG. 6, the clip 7 includes a pair of long clamping parts 71 and 72 which can be opened and closed relative to each other, and a hooked engaging part 73 for holding the pair of clamping parts 71 and 72 in a closed state. The pair of clamping parts 71 and 72 have a structure in which one of their opposed surfaces is formed with a projection 71A, whereas the other is formed with a recess 72A, so that the packaging member 6 clamped therebetween can be sealed. This ensures that even if oxygen is contained in the inside of the second outer package part 62, the inside of the first outer package part 61 can be kept in a deoxygenated state. Incidentally, the form of the clip 7 is not restricted to the just-mentioned form. In addition, the portion between the first outer package part 61 and the second outer package part 62 may be sealed by heat sealing (fusing) the packaging member 6, instead of using the clip 7.

Besides, the packaging member 6 is formed with a notch or notches 63 (see FIG. 3) in an end portion thereof, so that the packaging member 6 can be easily opened by tearing it up, starting from the notch 63, at the time of use.

The transport tube 3 for transporting the oxygen carrier is connected at its one end with a hollow needle 31 (see FIG. 1). When the hollow needle 31 is made to pierce the rubber element 27 of the housing pack 21, the transport tube 3 is made to communicate with the inside of the housing pack 21. The other end of the transport tube 3 is connected to the pump 4, so that the oxygen carrier in the inside of the housing pack 21 can be transported through the transport tube 3 to the pump 4.

Incidentally, it is more preferable that the transport tube 3 also has gas barrier properties.

The pump 4 is, for example, a tubular pump, which includes a rotor 41 capable of being rotated by a drive source such as a motor, rollers 42 rotatably fixed on the circumference of the rotor 41, and an elastic tube 43 located on the outer circumference of the rotor 41. When the rotor 41 is rotated, a roller 42 is moved while flattening one point of the elastic tube 43, whereby the fluid in the inside of the elastic tube 43 is pressed out. Then, the flattened part of the elastic tube 43 returns into its original shape by the restoring force of the elastic tube 43, to generate a vacuum inside the elastic tube 43, whereby the oxygen carrier suspension is sucked from the transport tube 3 into the elastic tube 43. Further, by the rotation of the rollers 42, the oxygen carrier suspension is pressurized and transported.

Incidentally, a method may also be adopted in which the transport tube 3 itself is attached to the rotor 41 of the pump 4 (tubular pump), and the oxygen carrier suspension is pressurized and transported by operating the rollers 42.

The pump 4 is so configured that the infusion speed and infusion quantity of the oxygen carrier being delivered can be controlled.

The oxygen carrier pressurized and transported by the pump 4 is introduced into a living organism through the microcatheter 5 (long element) shown in FIG. 7. The microcatheter 5 includes: a catheter body 51 which is formed therein with a lumen 52 and is formed with an opening 53 at its distal end; a hub part 54 connected to a proximal portion of the catheter body 51; and a thrombus-removing structure body which can be moved by sliding within the lumen 52. The structure body may be, for example, such a structure body as a retriever 58 (see FIG. 8).

The hub part 54 includes: an insertion hole 55 for permitting the thrombus-removing structure body 58 to be inserted into the lumen 52 of the catheter body 51 through a valve 57; and a port 56 to which a transport tube connected with the pump 4 is connected and through which the oxygen carrier is introduced from the pump 4 into the lumen 52.

In addition, the microcatheter may have a double-lumen structure for permitting individual passage of the oxygen carrier and the structure body 58.

The structure body 58 is a wire-shaped member formed at its distal end with a spiral structure part 59, and is used for removing a thrombus Z. The structure body 58 is formed from a superelastic material. The structure body 58 is so structured that the structure part 59 is elastically deformed into a straight shape and accommodated in the lumen 52 of the microcatheter 5 and that the structure part 59 returns into its original shape when protruded from the opening 53 of the microcatheter 5. Examples of the superelastic material applicable here include nickel-titanium alloys and copper-aluminum-manganese alloys. Further, in order to ensure good passage properties of the microcatheter and reduce injury to the blood vessel wall, the superelastic material may be coated with a hydrophilic material such as a block copolymer of dimethylacrylamide and glycidyl methacrylate. However, this is not restrictive.

The outside diameter of the catheter body 51 can be appropriately set according to the object into which the catheter body 51 is to be inserted. In the case of therapy of cerebral infarction, the outside diameter is preferably 0.5 to 2.0 mm, more preferably 0.5 to 1.5 mm.

The outside diameter of the wire constituting the structure body 58 is preferably smaller than the inside diameter of the lumen 52, to such an extent that a flow path for the oxygen carrier is provided inside the lumen 52 even when the structure body 58 is inserted in the lumen 52.

Or, in the case where the double-lumen structure is adopted, the outside diameter of the wire is preferably set according to the inside diameter of the lumen for passage of the structure body in such a manner that favorable passing properties can be obtained.

The use of the system 10 for delivering an oxygen carrier according to the present embodiment enables efficient supply of oxygen to a hypoxic site where blood flow is much decreased by occlusion or stenosis of a blood vessel in an ischemic lesion such as cerebral infarction, or a tissue put into a hypoxic state due to a defective development state of a blood vessel, such as cancer, or the like.

For instance, cerebral infarction as one of the applicable diseases may generally arise from occlusion of a cerebral artery due to a thrombus or embolus (ischemic cerebral infarction). The cerebral infarction is a state in which a brain tissue (brain parenchyma) in the flow region of a cerebral artery is exhibiting decay or necrosis due to dysfunction of blood flow in the brain, such as insufficient blood flow due to clogging or narrowing of a brain blood vessel, which in turn is caused when a peeled blood clot (thrombus or embolus) carried by blood flow enters into the cerebral blood vessel or when a thrombus Z is formed in the cerebral artery. Especially in the case of acute cerebral infarction, the possibility of recovery from the symptom can be much expected, by restarting the blood flow through dissolving the thrombus Z in the clogged brain blood vessel within a few hours from the onset of the cerebral infarction, or by sufficiently supplying blood (particularly, oxygen) into the blood vessels on the peripheral side relative to the clogged cerebral blood vessel. The use of the system 10 for delivering an oxygen carrier according to the present embodiment ensures that the oxygen carrier with a high oxygen carrying capacity can be selectively supplied to the brain tissue (brain parenchyma) where blood flow has been lowered (ischemic tissue). In addition, according to the system 10 for delivering an oxygen carrier according to this embodiment, the oxygen carrier transported after being oxygenated can, after arrival in the ischemic tissue X, release oxygen depending on the oxygen partial pressure in the tissue there. Therefore, oxygen can be efficiently supplied to the ischemic tissue X where oxygen is deficient.

It is also described a therapeutic method for ameliorating a hypoxic tissue, including the steps of: introducing a catheter into the hypoxic tissue, for example, into the ischemic tissue X beyond the thrombosed site Y causing the hypoxic state or to a position immediately on the proximal side of the thrombosed site Y; oxygenating an oxygen carrier which is in a deoxygenated state by an oxygenating part using the system 10 for delivering an oxygen carrier according to the present embodiment; and supplying the thus oxygenated oxygen carrier to the ischemic tissue X through the catheter.

In relation to therapy of a tumor tissue or a tissue which is in a hypoxic state due to deficient peripheral blood flow, there is provided a therapeutic method for ameliorating the hypoxic tissue, including the steps of: inserting the above-mentioned catheter into arterioles in the vicinity of the tumor or into peripheral arterioles; and supplying the above-mentioned oxygenated oxygen carrier into the arterioles through the catheter.

Incidentally, when utilized for ischemic lesions such as cerebral infarction or for diseases caused by peripheral circular insufficiency due to diabetes or the like, includes cure, healing, alleviation, relaxation, alteration, amelioration, improvement, recovery, betterment, and action, with regard to the patient's disease or symptom.

Further, in the therapy of a cancer, one example includes cure, healing, alleviation, relaxation, alteration, amelioration, improvement, recovery, betterment, and action, with regard to the disease or symptom of the patient with the cancer, by enhancing the sensitivity of the relevant site to radiation therapy or pharmacotherapy through bringing the tumor tissue which is in a hypoxic state to a hyperoxic state.

One example relates to a therapeutic method based on the supply of oxygen to the pathema tissues of the above-mentioned various diseases.

Here, as above-mentioned, cerebral infarction is generally classified into (i) cerebral thrombosis in which a blood vessel wall is pathologically altered by arterial sclerosis or the like to form a thrombus Z and to thereby obstruct the blood vessel, (ii) cerebral embolism in which a thrombus Z formed in some site in the body due to arterial sclerosis or a heart disease or the like is peeled and carried by blood flow to clog a blood vessel in the brain, and (iii) cerebral infarction caused by a decrease in cerebral blood flow or a reduction in the quantity of oxygen in the blood due to some disease. The method is suitable for all types of cerebral infarction. Particularly, the method is suitably applicable to acute cerebral infarction.

In relation to cardiac diseases, the method is suitably applicable to any of stenocardia arising from narrowing of a coronary artery due to arterial sclerosis and myocardial infarction in which a coronary artery is occluded with a thrombus Z due to arterial sclerosis.

Further, in relation to cancer, the method is suitably applicable to therapy of solid cancers of the type of poor sensitivity to radiation therapy or pharmacotherapy, in the cases where the therapy is to be applied after the quantity of oxygen in the cancer tissue has preliminarily been increased.

In addition, in relation to diseases where peripheral circular insufficiency is generated such as diabetes, the method is suitably applicable to therapy of arterial sclerosis of peripheral blood vessels and a tissue disorder due to a hypoxic state as a result of a decrease in blood flow caused by blood vessel constriction.

Now, taking cerebral infarction (particularly, acute cerebral infarction) as an example of a case of applicable diseases, a preferable mode of the method for treating a pathema caused by a hypoxic state by use of the system 10 for delivering an oxygen carrier will be described below, referring to the drawings.

First, after the clip 7 attached to the packaging member 6 of the housing 2 is detached, the packaging member 6 is opened by utilizing the notch 63 in the packaging member 6, and the housing pack 21 inside the packaging member 6 is taken out. Next, the housing pack 21 is pressed to cause delamination of the sealing part 28, whereby the oxygen carrier housing part 23 and the oxygen housing part 24 are made to communicate with each other, and the deoxygenated oxygen carrier and oxygen are mixed with each other, resulting in oxygenation. In this instance, the oxygenation takes place to achieve a high oxygen saturation, since the oxygen carrier is oxygenated at an oxygen partial pressure higher than the atmospheric oxygen partial pressure (about 150 mmHg). Thereafter, the housing pack 21 is hung on the hook J by utilizing the hanging hole 26 (see FIG. 1).

Subsequently, the hollow needle 31 of the transport tube 3 is used to pierce the rubber element 27 of the housing pack 21, thereby transporting the oxygen carrier to the pump 4 through the transport tube 3. This results in the oxygenated oxygen carrier being supplied into the microcatheter 5 with pressurization by the pump 4.

Then, as shown in FIG. 2, a guiding catheter 8 (for example, 2 mm in diameter) equipped with a balloon 81 is inserted through a femoral artery, a radial artery or a brachial artery , and is guided into an internal carotid artery or into the vicinity of the thrombosed site Y under radioscopy. Next, the catheter body 51 of the microcatheter 5 with a smaller diameter (for example, 0.5 mm in diameter) is inserted through the guiding catheter 8 to reach the ischemic tissue X beyond the thrombosed site Y (see FIG. 9). Thereafter, the pump 4 is operated to supply the oxygenated oxygen carrier to the ischemic tissue X through the microcatheter 5. Since the oxygen partial pressure in the ischemic tissue X is low (for example, about 2 to 40 mmHg), oxygen is efficiently supplied from the oxygen carrier to the ischemic tissue X. According to this method, oxygen can be sufficiently and selectively supplied to the brain tissue (brain parenchyma) where blood flow has been decreased (oxygen has been deficient), within a short time from the onset of the cerebral infarction. Consequently, recovery from the symptom can be greatly expected.

Thereafter, as shown in FIG. 10, the structure body 58 is inserted into the lumen 52 of the catheter body 51, and is protruded from the opening 53 of the microcatheter 5, whereby the spiral structure part 59 is restored into its original shape in the ischemic tissue X. Incidentally, it is preferable that the supply of the oxygen carrier is continued even during the procedure using the structure body 58. However, the supply may be stopped by stopping the pump 4, depending on the situation.

Then, as shown in FIG. 11, the structure body 58 is pulled backward together with the catheter body 51, whereby the thrombus Z is entangled with the structure part 59 of the structure body 58. In this condition, the balloon 81 attached to the guiding catheter 8 is inflated for controlling the blood flow, and the structure body 58 is further pulled backward, as it is, together with the catheter body 51, whereby the thrombus Z is recovered into the inside of the guiding catheter 8. Thereafter, the balloon 81 is deflated, and the guiding catheter 8 is pulled out of the artery in which it has been inserted. In this way, the procedure is completed.

Incidentally, the structure body 58 may not necessarily be provided. For example, unless the thrombus Z causes total occlusion of the blood vessel, the opening 53 of the microcatheter 5 may be disposed at a position immediately on the proximal side of the thrombosed site Y, instead of being disposed in a position beyond the thrombosed site Y. In this case, the oxygen carrier can be sent into the ischemic tissue X (which is the target site) through a gap or gaps in the thrombosed site Y. Particularly, a liposome encapsulated hemoglobin (LEH) type oxygen carrier has an outside diameter of about 200 nm, which is about one sixtieth of the outside diameter of red blood cells. Moreover, a hemoglobin solution type oxygen carrier is further smaller than the liposome encapsulated hemoglobin (LEH) type oxygen carrier. Therefore, the oxygen carrier can be efficiently fed into the ischemic tissue X (which is the target site) through the narrow gap or gaps.

In the above-mentioned therapeutic method, the step of oxygenating the oxygen carrier can be carried out extremely easily. Therefore, the oxygenating step may be performed after the arrival of the microcatheter 5 in the ischemic tissue X, particularly, immediately upon its arrival. This ensures that the oxygen carrier can display its oxygen carrying capacity as effectively as possible, so that a larger amount of oxygen can be supplied to the ischemic tissue X. Incidentally, the period after the arrival of the microcatheter 5 in the ischemic tissue X until the start of the supply of the oxygen carrier is preferably as short as possible. Ordinarily, this period is preferably not more than 60 minutes; in view of the oxygen carrying capacity and operability, the period is particularly preferably in the range of 10 to 30 minutes.

According to the system 10 for delivering an oxygen carrier in the first embodiment, the oxygen carrier can be delivered selectively and directly to the target site through the microcatheter (long element), and dissociation of oxygen from the oxygen carrier during the administration can be minimized. Consequently, the oxygen carrying capacity can be displayed as effectively as possible.

In addition, since the oxygen housing part 24 is provided in the housing pack 21, oxygenation of the oxygen carrier can be easily effected by only the operation of causing the oxygen housing part 24 and the oxygen carrier housing part 23 to communicate with each other through delamination of the sealing part 28 formed in the housing pack 21. Therefore, the oxygen carrier can be positively oxygenated to a high oxygen saturation, immediately before delivery into a living organism and while maintaining the sterile state of the oxygen carrier.

Besides, since the housing pack 21 is preserved in the state of being covered with the packaging member 6 formed from an oxygen-impermeable material, the oxygen carrier can be stored for a long time while restraining the change thereof into a methemoglobin type form.

In addition, according to the housing 2 (housing for oxygen carrier) in the first embodiment, it is possible, by simply effecting delamination of the sealing part 28, to cause the oxygen carrier housing part 23 and the oxygen housing part 24 to communicate with each other. Therefore, the oxygen carrier stored in the deoxygenated state can be oxygenated easily and rapidly. This ensures that the oxygen carrier can be positively oxygenated to a high oxygen saturation, immediately before the delivery into the living organism and while maintaining the sterile state of the oxygen carrier. Accordingly, the oxygen carrier can be stored in the deoxygenated state, until immediately before the delivery. Consequently, the oxygen carrier can be oxygenated while restraining, as securely as possible, the change thereof into a methemoglobin type form during storage thereof.

Besides, the packaging member 6 includes the first outer package part 61 covering the oxygen carrier housing part 23 in a sealing manner, the second outer package part 62 covering the oxygen housing part 24 in a sealing manner, and the clip 7 (sealing part) by which the inside space of the first outer package part 61 and the inside space of the second outer package part 62 are isolated from each other. Therefore, the oxygen carrier housing part 23 and the oxygen housing part 24 are sealed individually, and the deoxygenated state of the oxygen carrier in the oxygen carrier housing part 23 can be favorably maintained. In other words, although oxygen is contained in the inside of the second outer package part 62 sealing the oxygen housing part 24, the isolation between the first outer package part 61 and the second outer package part 62 prevents oxygen in the second outer package part 62 from moving into the first outer package part 61. This ensures that the deoxygenated state of the oxygen carrier in the oxygen carrier housing part 23 can be favorably maintained in the inside of the first outer package part 61.

In addition, since the deoxygenating agent 68 is housed in the inside of the first outer package part 61, oxygen present inside the first outer package part 61 is absorbed. Therefore, the deoxygenated state of the oxygen carrier inside the oxygen carrier housing part 23 can be maintained in a favorable manner.

Besides, the oxygen housing part 24 has a capacity (housing volume) sufficient for housing oxygen that is necessary for wholly(entirely) oxygenating the oxygen carrier housed in the oxygen carrier housing part 23. This ensures that the entirety of the oxygen carrier housed in the oxygen carrier housing part 23 can be oxygenated to a high oxygen saturation.

Incidentally, the configuration for supplying oxygen to the oxygen carrier in the housing pack 21 (housing 2) is not restricted to the mode in which the oxygen housing part 24 is provided in the housing pack 21. FIG. 12 shows a housing 9 for an oxygen carrier according to a modification of the present embodiment, in which oxygen can be externally injected into an oxygen housing part 24 of the housing 9 for an oxygen carrier. Incidentally, the parts having the same or equivalent functions to those in the above-described present embodiment are denoted by the same reference symbols as used above, and descriptions of the parts will be omitted.

An injection tube 91 (injection part) for injecting oxygen communicates with the oxygen housing part 24 of the housing 9 for an oxygen carrier, penetrates a packaging member 6 while maintaining the sealed state of the packaging member 6, and protrudes to the exterior. A cap 92 is attached to the protruding-side end of the injection tube 91. A known sterilizing filter 93 and a known easily breakable communicating part 94, which has a communicating pipe closed at one end thereof and is opened when part of the pipe is broken, are provided at intermediate portions of the injection tube 91.

The easily breakable communicating part 94 may be any communicating part having a sealing part that is provided so as to seal a flow path and that is broken under an external force, to release the sealed state of the flow path, thereby providing fluidic communication. For example, CLICK CHIP (trademark) (produced by Terumo Corporation) can be used as the easily breakable communicating part 94. The sealing part of CLICK CHIP has a tubular body which is closed at its one end and is provided so as to seal a flow path of a tube or the like. A thin-walled brittle breakable portion is formed at an outer circumference of the tubular body. When the breakable portion is externally bent together with the tube by fingers or the like, the flow path is put into a patent state.

As the sterilizing filter 93, use can be made of a hydrophobic filter having a pore diameter so as to prevent passage therethrough of bacteria, specifically a pore diameter of not more than 0.6 micrometer, preferably not more than 0.45 micrometer, and more preferably not more than 0.2 micrometer. As the hydrophobic filter, those formed from a hydrophobic resin such as polytetrafluoroethylene and polypropylene can be used. The sterilizing filter 93 is not restricted to the just-mentioned ones, insofar as it can trap bacteria when oxygen is injected into the oxygen carrier housing part.

The sterilizing filter 93 and the easily breakable communicating part 94 are sealed inside a third outer package part 64, which is formed inside the packaging member 6 by heat sealing (fusing) or the like so as to be isolated from the first outer package part 61 and the second outer package part 62. When the housing 9 for an oxygen carrier is stored, oxygen is not housed in the oxygen housing part 24. At the time of using the housing 9 for an oxygen carrier as above-mentioned, the third outer package part 64 is first opened, and the easily breakable communicating part 94 is broken to bring the injection tube 91 into a patent state. Next, the cap 92 is detached, oxygen is injected through the injection tube 91 by a syringe or the like, in an amount sufficient for wholly oxygenating the hemoglobin contained in the oxygen carrier housed in the oxygen carrier housing part 23. The oxygen passes through the sterilizing filter 93 to be housed in the oxygen housing part 24 in a sterilized state. Thereafter, the injection tube 24 is closed by pinching it with forceps or by bending it, and the injection tube 91 is closed by attaching the cap 92. Then, like in the first embodiment, the clip 7 is detached to cause delamination of the sealing part 28, whereby the oxygen carrier housing part 23 and the oxygen housing part 24 are let communicate with each other, resulting in oxygenation of the oxygen carrier.

According to the housing 9 for an oxygen carrier in this modification as above-mentioned, oxygen is not housed in the oxygen housing part 24 during storage. Therefore, mixing of oxygen into the oxygen carrier housing part 23 during storage can be restrained more securely. Incidentally, to the injection tube 91, other structure or structures such as a check valve may further be added.

In addition, at least one of the oxygen carrier housing part 23 and the oxygen housing part 24 may be formed from an oxygen-impermeable material. This ensures that the part of the housing pack 21 that is formed from an oxygen-impermeable material may not necessarily need to covered with further oxygen-impermeable material. Besides, an easily breakable communicating part can be used as a sealing part by which the oxygen carrier housing part 23 and the oxygen housing part 24 are sealed in such a manner that they can be made to communicate with each other. In addition, as the oxygen housing part, a capsule-shaped member in which oxygen is contained and which releases the oxygen into the inside of the oxygen carrier housing part 23 by being broken under an external pressure application may be used in the inside of the oxygen carrier housing part 23. Incidentally, in the case of using the easily breakable communicating part or the capsule-shaped member, a configuration is adopted to ensure that the broken member will not flow out into the transport tube 3. Besides, the packaging member 6 may be provided with a structure in which, for example, a part with a varied thickness or rigidity is provided in a straight form so as to guide the direction of opening (tearing-up) that starts from the notch 63.

A system 100 for delivering an oxygen carrier according to an example differs from that according to the first embodiment, in the means for oxygenating a deoxygenated oxygen carrier. Incidentally, the parts with the same or equivalent functions to those in the first embodiment are denoted by the same reference symbols as used above, and descriptions of the parts will be omitted.

As shown in FIG. 13, the system 100 for delivering an oxygen carrier includes: a housing 101 for housing and preserving a deoxygenated oxygen carrier; a transport tube 3 for transporting the oxygen carrier from a housing pack 110; an oxygenation device 120 for an oxygen carrier by which the deoxygenated oxygen carrier being transported is oxygenated in an aseptic condition; a pump 4 for pressurizing and feeding the oxygen carrier; and a microcatheter 5 (long element) by which the pressurized oxygen carrier is guided into a living organism. Thus, unlike in the first embodiment, the oxygen carrier is not oxygenated in the housing pack 110. Instead, oxygenation of the oxygen carrier is performed by the oxygenation device 120 for an oxygen carrier, after the deoxygenated oxygen carrier is transported out from the housing pack 110 and before the oxygen carrier is supplied into the microcatheter 5.

The housing 101 is a member in which the deoxygenated oxygen carrier is housed while being kept in the deoxygenated state for long-term preservation. The housing 101 includes the housing pack 110 in which the oxygen carrier is actually housed, and a packaging member 6 covering the housing pack 110. The housing pack 110 is formed from an oxygen-permeable film-shaped material, and is preserved in the state of being sealed with the oxygen-impermeable packaging member 6. However, the housing pack 110 itself may be formed from an oxygen-impermeable material.

As shown in FIG. 14, the housing pack 110 includes an oxygen carrier housing part 111 in which the oxygen carrier and the like are housed, and a tubular body 25 which communicates with the oxygen carrier housing part 111 and serves for transporting out the oxygen carrier. The oxygen carrier housing part 111 is formed from an oxygen-permeable film-shaped material in such a manner as to have a space therein. The housing pack 110 is formed, in its edge portion opposite to the tubular body 25, with a hanging hole 26 by which it is hung on a hook J when put to use.

The oxygen carrier housing part 111 is formed, for example, from polyethylene (PE), but the material is not restricted to polyethylene insofar as it is permeable to oxygen. In addition, the oxygen-permeable material may be provided at only part of the oxygen carrier housing part 111.

The tubular body 25 is heat sealed (fused) or adhered to the film-shaped material so as to communicate with the oxygen carrier housing part 111. The tubular body 25 is disposed so that its one end protrudes from the oxygen carrier housing part 111, and a rubber element 27 for sealing the inside space of the oxygen carrier housing part 111 is provided inside the protruding-side end portion of the tubular body 25.

In the inside of the packaging member 6, a deoxygenating agent 68 and an oxygen detecting agent 69 for detection of oxygen by color tone are sealed, together with the oxygen carrier housing part 111. Therefore, hemoglobin contained in the oxygen carrier in the oxygen carrier housing part 111 is deoxygenated by the deoxygenating agent 68 through the oxygen-permeable film constituting the oxygen carrier housing part 111, after being packaged with the packaging member 6. Since the packaging member 6 is impermeable to oxygen, the oxygen carrier is maintained in the deoxygenated state, and the deoxygenated state can be confirmed by visual inspection based on the oxygen detecting agent 69.

The transport tube 3 for transporting the oxygen carrier is allowed to communicate with the inside of the housing pack 110, by a method in which a hollow needle 31 connected to one end of the transport tube 3 is made to pierce the rubber element 27 of the housing pack 110 (see FIG. 13). The other end of the transport tube 3 is connected to the oxygenation device 120 for an oxygen carrier, so that the oxygen carrier inside the housing pack 110 can be transported through the transport tube 3 to the oxygenation device 120 for an oxygen carrier.

As shown in FIG. 15, the oxygenation device 120 (oxygenation part) for an oxygen carrier includes an oxygen-permeable tube 121 (oxygen-permeable membrane) connected so as to communicate with the transport tube 3, and an oxygen supply chamber 122 provided so as to cover the oxygen-permeable tube 121. The oxygen supply chamber 122 is supplied with oxygen through an oxygen supply port 123, and surplus oxygen is discharged via an oxygen discharge port 124. The oxygen-permeable tube 121 inlcudes a flow part provided therein with a flow path 121A in which the oxygen carrier is allowed to flow. In addition, the oxygen-permeable tube 121 permits oxygen in the oxygen supply chamber 122 to permeate therethrough to the inside thereof, whereby the oxygen carrier flowing in the flow path 121A can be oxygenated.

The total amount of oxygen supplied into the oxygen supply chamber 122 is preferably an amount sufficient for wholly oxygenating the hemoglobin in the oxygen carrier housed in the oxygen carrier housing part 111. Therefore, in the case where for example 100 ml of an oxygen carrier suspension is housed in the oxygen carrier housing part 111 and where 6 g of hemoglobin is contained in the suspension, about 8 ml of oxygen is needed since the amount of oxygen necessary per 1 g of hemoglobin is about 1.35 ml. In view of this, oxygen is supplied into the oxygen supply chamber 122 in an amount of not less than about 8 ml. In order to enhance the oxygen saturation of the oxygen carrier, it is preferable for oxygen supplied into the oxygen supply chamber 122 to have an oxygen partial pressure higher than the atmospheric oxygen partial pressure (about 150 mmHg). It is preferable to use pure oxygen (oxygen concentration: 100%), but this is not restrictive.

For the oxygen-permeable tube 121, there can be used, for example, a hydrophobic porous film obtained by forming a film of polypropylene (PP), polytetrafluoroethylene (PTFE), polyethylene (PE), polyvinyl chloride, polyvinyl acetate, polyurethane, or the like with microscopic through-holes. Or, alternately, gas exchange membranes commonly used for artificial hearts and lungs or the like, such as thin films of materials having a high oxygen permeability, such as silicone rubber, can be applied.

The oxygenation device 120 for an oxygen carrier has an oxygen gas exchange capacity of 0.04 to 10.0 cc/min, preferably 0.1 to 8.0 cc/min, and more preferably 0.2 to 8.0 cc/min. In addition, the area of the oxygen-permeable tube for the oxygen gas exchange, which varies depending on the oxygen gas exchange capacity of the material used, is 0.4 to 800 cm², preferably 2 to 400 cm², and more preferably 5 to 200 cm². In these ranges, operability similar to that of an ordinary infusion set can be obtained.

At the time of using the system 100 for delivering an oxygen carrier, the packaging member 6 is first opened by utilizing a notch 63 in the packaging member 6, and the housing pack 110 inside the packaging member 6 is taken out. Thereafter, the housing pack 110 in which the oxygen carrier is housed in the as-deoxygenated state is hung from the hook J by utilizing the hanging hole 26, the hollow needle 31 of the transport tube 3 is made to pierce the rubber element 27 of the housing pack 110, and the oxygen carrier is transported through the transport tube 3 into the oxygenation device 120 for an oxygen carrier. In the oxygenating device 120 for an oxygen carrier, the oxygen carrier in the oxygen-permeable tube 121 is oxygenated to a high oxygen saturation by the oxygen in the oxygen supply chamber 122 through the oxygen-permeable tube 121, since the oxygen partial pressure in the oxygen supply chamber 122 is higher than the atmospheric oxygen partial pressure. The oxygenated oxygen carrier is transported to the pump 4, and can be supplied to the microcatheter 5 under pressurization by the pump 4. Incidentally, the subsequent procedure is the same as in the first embodiment, and, therefore, description thereof is omitted here.

According to the system 100 for delivering an oxygen carrier, the oxygen carrier is oxygenated immediately before delivery into a living organism. Therefore, the oxygen carrying capacity can be exhibited as effectively as possible. In addition, where a configuration is adopted in which the oxygen-impermeable properties of the housing pack 110 can be maintained even during use, the change of the oxygen carrier into a methemoglobin type form does not occur before transport of the oxygen carrier out of the housing pack 110. Therefore, the housing pack 110 can be used for a long time. That is, where the housing pack 110 is formed from an oxygen-permeable material, the change of the oxygen carrier into a methemoglobin type form starts after the housing pack 110 is taken out of the packaging member 6; therefore, the housing pack 110 has to be used within a few hours. If the oxygen-impermeable properties of the housing pack 110 can be maintained even during use, on the other hand, it is then unnecessary to take into account the change of the oxygen carrier into a methemoglobin type form in the housing pack 110; accordingly, the housing pack 110 can be used for a long time even after taken out of the packaging member 6. Incidentally, a configuration in which the oxygen-impermeable properties of the housing pack 110 can be maintained even during use can be realized by forming the housing pack 110 itself from an oxygen-impermeable material, or by configuring the housing pack 110 to be usable in the state of being enveloped with an oxygen-impermeable material.

According to the oxygenation device 120 for an oxygen carrier, the flow path 121A into which the deoxygenated oxygen carrier flows is formed inside the oxygen-permeable tube 121 in the state of being partitioned from the oxygen supply chamber 122. Therefore, the oxygen carrier thus flowing in can be continuously and efficiently oxygenated through the oxygen-permeable tube 121 and in a short time. In addition, the oxygen carrier can be oxygenated immediately before delivery into an ischemic tissue X (hypoxic tissue). Therefore, the oxygen carrying capacity can be exhibited as effectively as possible, and oxygen can be supplied into the ischemic tissue X in a large quantity. Besides, it is unnecessary to positively oxygenate the oxygen carrier in the housing pack 110. Therefore, the change of the oxygen carrier into a methemoglobin type form in the housing pack 110 would not easily proceed, so that one housing pack 110 can be used for a long time.

In addition, since the oxygen supply chamber 122 is provided with the oxygen supply port 123 and the oxygen discharge port 124, continuous supply of oxygen can be easily realized. As a result, it is easy to control the oxygen partial pressure in the oxygen supply chamber 122, and it is possible to control the oxygen saturation of the oxygen carrier.

Incidentally, the oxygenation device for an oxygen carrier is not restricted to the configuration shown in FIG. 15. FIG. 16 shows a modification of the oxygenation device for an oxygen carrier, in which an oxygen-permeable tube 125 is formed in a zigzag pattern, for increasing the area of permeation of oxygen. Or, alternatively, the oxygen-permeable tube may be formed in a spiral pattern. In addition, as in a further example of the oxygenation device for an oxygen carrier as shown in FIG. 17, a plurality of oxygen-permeable tubes 126 (inclusive of hollow fiber, for example) may be used to constitute the device, for further increasing the area of permeation of oxygen. Besides, the oxygen-permeable membrane may be in other structure (not shown) than a tubular structure, insofar as the oxygen supply chamber and the flow path of the flowing part in which the oxygen carrier flows are partitioned from each other by the oxygen-permeable membrane.

In addition, as a still further example of the oxygenation device for an oxygen carrier, a configuration as shown in FIG. 18 may be adopted in which an oxygenation device 130 for an oxygen carrier includes an oxygen-permeable tube 131 and an oxygen supply chamber 132, and oxygen is sealed in the oxygen supply chamber 132 in a fixed amount, instead of constantly flowing in the oxygen supply chamber 132. Oxygen is preferably sealed in the oxygen supply chamber 132 in an amount not less than the amount necessary for oxygenation of the oxygen carrier in the housing pack 110, but this is not restrictive. In this case, as the oxygen in the oxygen supply chamber 132 is dissolved in the oxygen carrier, the amount of oxygen in the oxygen supply chamber 132 decreases. In view of this, it is preferable that the capacity (housing volume) of the oxygen supply chamber 132 is variable according to a variation in the amount of oxygen in the oxygen supply chamber 132. Specifically, where the oxygen supply chamber 132 is formed from a low-rigidity material into a bellows-like shape, it can be ensured that the oxygen supply chamber 132 is reduced or enlarged in volume accordingly as the amount of oxygen in the inside thereof decreases or increases. Such a configuration ensures that even if the amount of oxygen in the oxygen supply chamber 132 is decreased attendant on the oxygenation of the oxygen carrier, the oxygen supply chamber 132 deforms so that the oxygen partial pressure is automatically kept constant, whereby the oxygen saturation of the oxygen carrier can be automatically controlled. Where a configuration is adopted in which oxygen is sealed in such an oxygen supply chamber 132, the system can be used even in a place where an oxygen supply source is absent. Examples of the material applicable to form the oxygen supply chamber 132 include oxygen barrier resin films such as films of EVOH (ethylene-vinyl alcohol acetate copolymer), O-PVA (biaxially oriented polyvinyl alcohol), PVDC (vinylidene chloride copolymer), etc., and barrier films obtained by coating a film of PET (polyethylene terephthalate) or the like with a thin film of an inorganic oxide such as silicon oxide, alumina, etc. Incidentally, if the volume of the oxygen supply chamber 132 is sufficiently larger than the necessary amount of oxygen, the oxygen supply chamber 132 may not necessarily be deformable. Besides, the structure for permitting variations in the volume of the oxygen supply chamber 132 is not restricted to the above-mentioned bellows-like structure.

Another system 200 for delivering an oxygen carrier differs from that according to the first embodiment, in the means for oxygenating a deoxygenated oxygen carrier. Incidentally, the parts with the same or equivalent functions to those in the first embodiment are denoted by the same reference symbols as used above, and descriptions of the parts will be omitted.

As shown in FIG. 19, the system 200 for delivering an oxygen carrier includes: a housing 201 for an oxygen carrier in which to house and preserve an oxygen carrier; a transport tube 3 for transporting the oxygen carrier from the housing 201 for an oxygen carrier; a pump 4 for pressurizing the oxygen carrier transported through the transport tube 3; and a microcatheter 5 (long element) through which the pressurized oxygen carrier is guided into a living organism.

As shown in FIGS. 20 to 22, the housing 201 for an oxygen carrier is a member in which the oxygen carrier is housed while being kept in the deoxygenated state for the purpose of long-term preservation. The housing 201 for an oxygen carrier includes a housing pack 210 in which the oxygen carrier is actually housed, and a packaging member 230 covering the housing pack 210.

The housing pack 210 includes an oxygen carrier housing part 211 in which the oxygen carrier and the like are housed, an injection tube 212 (injection part) through which oxygen can be injected, and a tubular body 25 which communicates with the oxygen carrier housing part 211 and serves for transporting out the oxygen carrier. A cap 213 is attached to an end of the injection tube 212. A known sterilizing filter 214 and a known easily breakable communicating part 215, which has a communicating pipe closed at its one end and is opened when part of the pipe is broken, are provided at intermediate portions of the injection tube 212. The easily breakable communicating part 215 may be any communicating part having a sealing part that is provided so as to seal a flow path and that is broken under an external force to release the sealed state of the flow path, thereby providing fluidic communication. For example, CLICK CHIP (trademark) (produced by Terumo Corporation) can be used as the easily breakable communicating part 215. The sealing part of CLICK CHIP has a tubular body which is closed at its one end and is provided so as to seal a flow path of a tube or the like. A thin-walled brittle breakable portion is formed at an outer circumference of the tubular body. When the breakable portion is externally bent together with the tube by fingers or the like, the flow path is put into a patent state. Besides, to the injection tube 212, other structure or structures such as a check valve may be further added.

As the sterilizing filter 214, use can be made of a hydrophobic filter having such a pore diameter as to prevent passage therethrough of bacteria, specifically a pore diameter of not more than 0.6 micrometer, preferably not more than 0.45 micrometer, and more preferably not more than 0.2 micrometer. As the hydrophobic filter, those formed from a hydrophobic resin such as polytetrafluoroethylene and polypropylene can be used. The sterilizing filter 214 is not restricted to the just-mentioned ones, insofar as it can trap bacteria when oxygen is injected into the oxygen carrier housing part.

The oxygen carrier housing part 211 is formed from an oxygen-permeable film-shaped material so as to have a space in the inside thereof. The housing pack 210 is formed, in its edge portion opposite to the tubular body 25, with a hanging hole 26 by which it is hung on a hook J when put to use.

The oxygen carrier housing part 211 is formed, for example, from polyethylene (PE), but the material is not restricted to polyethylene, insofar as it is permeable to oxygen. Besides, the oxygen-permeable material may be provided only in part of the oxygen carrier housing part 211.

The tubular body 25 is heat sealed (fused) or adhered to the film-shaped material so as to communicate with the oxygen carrier housing part 211. The tubular body 25 is disposed so that one end thereof protrudes from the oxygen carrier housing part 211. Inside the protruding-side end portion of the tubular body 25, there is provided a rubber element 27 for sealing the inside space of the oxygen carrier housing part 211.

The packaging member 230, which envelopes the whole body of the housing pack 210, is formed from an oxygen-impermeable film-shaped material. The material constituting the packaging member 230 is desirably transparent so that the inside thereof can be visually checked. The oxygen-impermeable and light-transmitting material for the packaging member 230 include films having an oxygen barrier resin layer of EVOH (ethylene-vinyl alcohol acetate copolymer), O-PVA (biaxially oriented polyvinyl alcohol), PVDC (vinylidene chloride copolymer) or the like, and films having a barrier layer obtained by coating a film of PET (polyethylene terephthalate) or the like with a thin film of an inorganic oxide such as silicon oxide, alumina, etc. by vapor deposition or the like. However, the material for the packaging member 230 is not restricted to these films, so long as the material is impermeable to oxygen.

As shown in FIGS. 20 and 22, the packaging member 230 includes: a first outer package part 231 covering the oxygen carrier housing part 211 of the housing pack 210; a second outer package part 232 covering the injection tube 212 (injection part) together with the sterilizing filter 214 and the easily breakable communicating part 215; and a sealing part 233 sealing a portion between the first outer package part 231 and the second outer package part 232.

The sealing part 233 is also formed in secure contact with the outer surface of the injection tube 212, for example, by heat sealing (fusing) the film-shaped resin material constituting the packaging member 230 under controlled temperature and pressure.

In each of the inside of the first outer package part 231 and the inside of the second outer package part 232, a deoxygenating agent 68 and an oxygen detecting agent 69 for detection of oxygen by color tone are sealed. Therefore, the hemoglobin contained in the oxygen carrier in the oxygen carrier housing part 211 is deoxygenated by the deoxygenating agent 68 in the first outer package part 231 through the oxygen-permeable film of the oxygen carrier housing part 211. In addition, the inside of the second outer package part 232 is also deoxygenated, whereby penetration of oxygen into the oxygen carrier housing part 211 through the injection tube 212 is restrained. Further, since the packaging member 230 is impermeable to oxygen, the oxygen carrier is maintained in the deoxygenated state, and the deoxygenated state can be confirmed by visual inspection based on the oxygen detecting agent 69.

Of the packaging member 230, the first outer package part 231 is formed with a first notch or notches 234 in edge portions thereof. Therefore, the first outer package part 231 can be easily opened by tearing, starting from the first notch 234. Of the packaging member 230, in addition, the second outer package part 232 is formed with a second notch or notches 235 in edge portions thereof. Therefore, the second outer package part 232 can be easily opened by tearing, starting from the second notch 235.

The transport tube 3 for transporting the oxygen carrier is connected at its one end with a hollow needle 31. When the hollow needle 31 is made to pierce the rubber element 27 of the housing pack 210, the transport tube 3 is made to communicate with the inside of the housing pack 210 (see FIG. 19). The other end of the transport tube 3 is connected to the pump 4, so that the oxygen carrier in the inside of the housing pack 210 can be transported through the transport tube 3 to the pump 4.

At the time of using the system 200 for delivering an oxygen carrier, as shown in FIG. 23, the second outer package part 232 is first opened by tearing the housing 201 for an oxygen carrier, starting from the second notch 235, whereby the injection tube 212 is exposed. Incidentally, even after the second outer package part 232 is opened, the oxygen carrier housing part 211 is kept in the sealed state by the first outer package part 231, so that the deoxygenated state of the oxygen carrier is maintained. Therefore, the housing 201 for an oxygen carrier can be stored in this condition for a predetermined period of time.

Next, the easily breakable communicating part 215 is broken to bring the injection tube 212 into a patent state. Then, the cap 213 is detached, and oxygen is injected into the oxygen carrier housing part 211 via the injection tube 212 by use of a syringe 240 (oxygen supply amount control part). The oxygen is housed into the oxygen carrier housing part 211 via the sterilizing filter 214 in a sterile state, and the oxygen carrier is oxygenated.

In the syringe 240, oxygen is contained in an amount sufficient for wholly oxygenating the hemoglobin in the oxygen carrier housed in the oxygen carrier housing part 211. Therefore, in an example of a case where 100 ml of an oxygen carrier suspension is housed in the oxygen carrier housing part 211 and where 6 g of hemoglobin is contained in the suspension, about 8 ml of oxygen is needed since the amount of oxygen necessary per 1 g of hemoglobin is about 1.35 ml. In view of this, oxygen is housed in the syringe 240 in an amount of not less than about 8 ml. In order to enhance the oxygen saturation of the oxygen carrier, it is preferable for the oxygen housed in the oxygen housing part 212 to have an oxygen partial pressure higher than the atmospheric oxygen partial pressure (about 150 mmHg). It is preferable to use pure oxygen (oxygen concentration: 100%), but this is not restrictive. The housing 201 for an oxygen carrier constitutes an oxygenation system for an oxygen carrier, together with the syringe 240 (oxygen supply amount control part).

After oxygen is injected into the oxygen carrier housing part 211 by the syringe 240, the injection tube 212 is closed by pinching the injection tube 212 with forceps or by bending the injection tube 212, and the injection tube 212 is closed by attaching the cap 213. Thereafter, the forceps is detached or the bent state is released.

Next, the first outer package part 231 is opened by utilizing the first notch 234, the sealing part 233 joined to the injection tube 212 is peeled and detached, and the packaging member 230 is removed from the housing pack 210. Thereafter, the housing pack 210 is hung on the hook J by utilizing the hanging hole 26 (see FIG. 19).

Subsequently, the hollow needle 31 of the transport tube 3 is made to pierce the rubber element 27 of the housing pack 210, whereby the oxygen carrier is transported through the transport tube 3 to the pump 4. This results in the oxygenated oxygen carrier being supplied to the microcatheter 5 under pressurization by the pump 4.

According to the housing 201 for an oxygen carrier, oxygen can be injected into the oxygen carrier housing part 211 via the injection tube 212. Therefore, the oxygen carrier stored in the deoxygenated state can be oxygenated easily and rapidly. This ensures that the oxygen carrier can be positively oxygenated to a high oxygen saturation while being kept in an aseptic condition, immediately before delivery into a living organism. Accordingly, the oxygen carrier can be stored in the deoxygenated state until immediately before the delivery. Consequently, the oxygen carrier can be oxygenated while restraining, as securely as possible, the change of the oxygen carrier into a methemoglobin type form during storage.

In addition, since the housing pack 210 is preserved while being covered (in a sealed manner) by the packaging member 230 formed from an oxygen-impermeable material, the oxygen carrier can be stored for a long time while restraining the change of the oxygen carrier into a methemoglobin type form.

Besides, the packaging member 230 includes the first outer package part 231 covering the oxygen carrier housing part 211 in a sealing manner, and the second outer package part 232 covering the injection tube 212 in the state of being isolated from the first outer package part 231. Therefore, even after the second outer package part 232 is opened for taking out the injection tube 212, the oxygen carrier housing part 211 is kept sealed with the first outer package part 231, so that the deoxygenated state of the oxygen carrier in the oxygen carrier housing part 211 can be maintained favorably.

In addition, since the injection tube 212 is equipped with the sterilizing filter 214, oxygen can be injected into the oxygen carrier housing part 211 in a sterile state.

Besides, the syringe 240 (oxygen supply amount control part) capable of controlling the amount of oxygen is connected to the housing 201 for an oxygen carrier through the injection tube 212 to constitute the oxygenation system for an oxygen carrier. Therefore, a desirable amount of oxygen can be appropriately injected into the inside of the housing 201 for an oxygen carrier, and the whole of the oxygen carrier housed in the oxygen carrier housing part 211 can be oxygenated to a high oxygen saturation.

In addition, as a modification, a configuration may be adopted in which structures varied in thickness or rigidity may be provided in straight forms in the packaging member 230 so as to guide the direction of opening (tearing-up) that starts from each of the first notch 234 and the second notch 235.

Besides, an other structure than the syringe 240 may be used as the oxygen supply amount control part constituting the oxygenation system for an oxygen carrier, together with the housing 201 for an oxygen carrier. FIG. 24 shows an oxygen supply amount control device 300 as a modification of the oxygen supply amount control part. The oxygen supply amount control device 300 includes: an oxygen supply tube 301 connected to an oxygen supply source; an expandable-and-contractible expansion part 303 supplied with oxygen from the oxygen supply tube 301 through an openable-and-closable first valve 302; and a discharge port 305 through which oxygen in the expansion part 303 is injected into the injection tube 212 of the housing pack 210 through an openable-and-closable second valve 304. The expansion part 303 is formed, for example, from an elastic body such as rubber and can contain a fixed amount of oxygen. At the time of injecting oxygen into the injection tube 212 by the oxygen supply amount control device 300, first, the first valve 302 is opened with the second valve 304 kept closed, whereby oxygen is supplied into the expansion part 303 from the oxygen supply tube 301. The expansion part 303 is expanded by the pressure of the oxygen supply source, and the expansion is stopped when a fixed amount of oxygen has been contained in the expansion part 303. Next, the first valve 302 is closed, the discharge port 305 is connected to the injection tube 212, and the second valve 304 is opened. As a result, the contracting force of the expansion part 303 causes oxygen in the expansion part 303 to be injected into the oxygen carrier housing part 211 through the injection tube 212. By such a configuration, oxygen can be supplied into the oxygen carrier housing part 211 in a fixed amount according to the size of the expansion part 303.

In addition, FIG. 25 shows an oxygen supply amount control device 400 as another modification of the oxygen supply amount control part. The oxygen supply amount control device 400 includes: an oxygen supply tube 401 connected to an oxygen supply source; a pump 402 supplied with oxygen through the oxygen supply tube 401; and a discharge port 403 through which oxygen from the pump 402 is injected into the injection tube 212 of the housing pack 210. The pump 402 is capable of controlling the flow rate of oxygen being supplied. At the time of injecting oxygen into the injection tube 212 by the oxygen supply amount control device 400, the discharge port 403 is connected to the injection tube 212, and the pump 402 is operated. The pump 402 is set so as to be stopped when a fixed amount of oxygen has been supplied. This ensures that a fixed amount of oxygen can be injected into the oxygen carrier housing part 211 through the injection tube 212.

The present invention is not restricted only to the above-described embodiments, and various modifications can be made within the technical thought of the invention by a person skilled in the art. Besides, a blood filter, a bubble-removing device, a temperature controller or the like may be provided in any position between the housing pack and the microcatheter. In addition, since the flow speed of the oxygen carrier is slower as compared with that in artificial heart and lung or the like, oxygenation of the oxygen carrier may be effected by a configuration in which a bubble-removing device or the like is provided to permit contact between the oxygen carrier and oxygen, without using the oxygen-permeable membrane.

Besides, the deoxygenation of the oxygen carrier is conducted for restraining the change of the oxygen carrier into a methemoglobin type form during storage. In this case, the oxygen carrier may not necessarily be deoxygenated completely, insofar as the change of the oxygen carrier into a methemoglobin type form during storage can be restrained. For instance, a condition where the deoxygenation of the oxygen carrier is incomplete is, naturally, also included in the condition where the oxygen carrier is deoxygenated.

Furthermore, the present application is based on Japanese Patent Application No. 2011-051943 filed on March 9, 2011, Japanese Patent Application No. 2011-051950 filed on March 9, 2011, Japanese Patent Application No. 2011-04842 filed on March 11, 2011, and Japanese Patent Application No. 2011-04843 filed on March 11, 2011.

### Explanation of Reference Symbols

2, 9, 101, 201 Housing,
5 Microcatheter (Long element),
6, 230 Packaging member,
7 Clip (Sealing part),
10, 100, 200 System for delivering oxygen carrier,
21, 210 Housing pack (Oxygenation part),
23, 111, 211 Oxygen carrier housing part,
24 Oxygen housing part,
28, 233 Sealing part,
240 Syringe (Oxygen supply amount control part),
51 Catheter body,
52 Lumen,
53 Opening,
61, 231 First outer package part,
62, 232 Second outer package part,
68 Deoxygenating agent,
91, 212 Injection tube (Injection part),
93, 214 Sterilizing filter,
300, 400 Oxygen supply amount control device (Oxygen supply amount control part),
120, 130 Oxygenation device for oxygen carrier (Oxygenation part),
121, 125, 126, 131 Oxygen-permeable tube (Oxygen-permeable membrane),
121A Flow path,
122, 132 Oxygen supply chamber,
123 Oxygen supply port,
124 Oxygen discharge port,
X Ischemic tissue,
Y Thrombosed site,
Z Thrombus.

## Claims

1. A system for delivering an oxygen carrier, comprising:
a housing (2) in which a hemoglobin-based oxygen carrier is housed in a deoxygenated state;
an oxygenation part (21) for oxygenating the deoxygenated oxygen carrier; and
a microcatheter (5) which can be inserted into a living organism and can release the oxygenated oxygen carrier through a lumen (52) that is formed inside thereof; and
**characterized in that**
the microcatheter (5) includes:
a catheter body (51) which is formed therein with the lumen (52) and is formed with an opening (53) at its distal end;
a hub part (54) connected to a proximal portion of the catheter body (51); and
a thrombus-removing structure body (58) which can be moved by sliding within the lumen (52).

2. The system for delivering an oxygen carrier according to claim 1, wherein the oxygenation part (21) is provided in a transport path for the oxygen carrier that is located between the housing (2) and the long element (5).

3. The system for delivering an oxygen carrier according to claim 1, wherein the oxygenation part (21) mixes oxygen into the oxygen carrier present in the inside of the housing (2).

4. The system for delivering an oxygen carrier according to any one of claims 1 to 3, wherein the housing (2) includes an oxygen-impermeable material for restraining the housed oxygen carrier from oxygenation by external oxygen.

## Patentansprüche

1. System zur Abgabe eines Sauerstoffträgers, mit:
einem Gehäuse (2), in dem ein Sauerstoffträger auf Hämoglobinbasis in einem desoxygenierten Zustand untergebracht ist;
einem Oxygenierungsteil (21) zur Oxygenierung des deoxygenierten Sauerstoffträgers; und
einem Mikrokatheter (5), der in einen lebenden Organismus eingeführt werden kann und den sauerstoffhaltigen Sauerstoffträger durch ein in seinem Inneren ausgebildetes Lumen (52) freisetzen kann; und
**dadurch gekennzeichnet, dass**
der Mikrokatheter (5) hat:
einen Katheterkörper (51), der mit dem Lumen (52) darin ausgebildet ist und an seinem distalen Ende mit einer Öffnung (53) ausgebildet ist;
ein Nabenteil (54), das mit einem proximalen Abschnitt des Katheterkörpers (51) verbunden ist; und
einen Körper (58) mit einer thrombusentfernenden Struktur, der durch Gleiten innerhalb des Lumens (52) bewegt werden kann.

2. System zur Abgabe eines Sauerstoffträgers nach Anspruch 1, wobei das Oxygenierungsteil (21) in einem Transportpfad für den Sauerstoffträger vorgesehen ist, der sich zwischen dem Gehäuse (2) und dem langen Element (5) befindet.

3. System zur Abgabe eines Sauerstoffträgers nach Anspruch 1, wobei das Oxygenierungsteil (21) Sauerstoff in den in dem Inneren des Gehäuses (2) vorhandenen Sauerstoffträger einmischt.

4. System zur Abgabe eines Sauerstoffträgers nach einem der Ansprüche 1 bis 3, wobei das Gehäuse (2) ein sauerstoffundurchlässiges Material enthält, um den aufgenommenen Sauerstoffträger von der Oxygenierung durch externen Sauerstoff abzuhalten.

## Revendications

1. Système pour distribuer un transporteur d'oxygène comprenant :
un boîtier (2) dans lequel un transporteur d'oxygène à base d'hémoglobine est logé dans un état désoxygéné ;
une partie d'oxygénation (21) pour oxygéner le transporteur d'oxygène désoxygéné ; et
un micro-cathéter (5) qui peut être inséré dans un organisme vivant et peut libérer le transporteur d'oxygène oxygéné à travers une lumière (52) qui est formée à l'intérieur de ce dernier ; et
**caractérisé en ce que** :
le micro-cathéter (5) comprend :
un corps de cathéter (51) qui est formé à l'intérieur de ce dernier avec une lumière (52) et est formé avec une ouverture (53) au niveau de son extrémité distale ;
une partie de raccord (54) raccordée à une partie proximale du corps de cathéter (51) ; et
un corps de structure d'extraction de thrombus (58) qui peut être déplacé par coulissement à l'intérieur de la lumière (52) .

2. Système pour distribuer un transporteur d'oxygène selon la revendication 1, dans lequel la partie d'oxygénation (21) est prévue dans une trajectoire de transport pour le transporteur d'oxygène qui est positionné entre le boîtier (2) et l'élément long (5).

3. Système pour distribuer un transporteur d'oxygène selon la revendication 1, dans lequel la partie d'oxygénation (21) mélange l'oxygène avec le transporteur d'oxygène présent à l'intérieur du boîtier (2).

4. Système pour distribuer un transporteur d'oxygène selon l'une quelconque des revendications 1 à 3, dans lequel le boîtier (2) comprend un matériau imperméable à l'oxygène pour empêcher l'oxygénation du transporteur d'oxygène logé, par l'oxygène externe.
